# EUROPEAN PATENT APPLICATION

(11) **EP 1 908 476 A1**
(43) Date of publication of application: **09.04.2008**
(21) Application number: 06020467.4
(22) Date of filing: 28.09.2006
(51) Int. Cl.: A61K 38/17, A61P 17/06

(54) **Galectin-2 for the treatment of inflammatory diseases of the skin**

(71) Applicant: Charité-Universitätsmedizin Berlin, 10117 Berlin (DE); Universitätsklinikum Münster, 48149 Münster (DE)
(72) Inventor: Sturm, Andreas, 14167 Berlin (DE); Dignass, Axel, 60431 Frankfurt/Main (DE); Loser, Karin, 48341 Altenberge (DE); Beissert, Stefan, 48143 Münster (DE)
(74) Representative: Engelhard, Markus

(57) **Abstract**

The present invention relates to novel uses of galectin-2.

SEQUENCE LISTING

<110>  Charite

<120>  Uses of Galectin-2

<130>  FB17753

<160>  2

<170>  PatentIn version 3.2

<210>  1
<211>  132
<212>  PRT
<213>  Homo sapiens

<400>  1

Met Thr Gly Glu Leu Glu Val Lys Asn Met Asp Met Lys Pro Gly Ser
1               5                   10                  15

Thr Leu Lys Ile Thr Gly Ser Ile Ala Asp Gly Thr Asp Gly Phe Val
            20                  25                  30

Ile Asn Leu Gly Gln Gly Thr Asp Lys Leu Asn Leu His Phe Asn Pro
        35                  40                  45

Arg Phe Ser Glu Ser Thr Ile Val Cys Asn Ser Leu Asp Gly Ser Asn
    50                  55                  60

Trp Gly Gln Glu Gln Arg Glu Asp His Leu Cys Phe Ser Pro Gly Ser
65                  70                  75                  80

Glu Val Lys Phe Thr Val Thr Phe Glu Ser Asp Lys Phe Lys Val Lys
                85                  90                  95

Leu Pro Asp Gly His Glu Leu Thr Phe Pro Asn Arg Leu Gly His Ser
            100                 105                 110

His Leu Ser Tyr Leu Ser Val Arg Gly Gly Phe Asn Met Ser Ser Phe
        115                 120                 125

Lys Leu Lys Glu
    130

<210>  2
<211>  130
<212>  PRT
<213>  Rattus norvegicus

<400>  2

Met Ser Glu Lys Phe Glu Val Thr Asn Leu Asn Met Lys Ser Gly Met
1               5                   10                  15

Ser Leu Lys Ile Lys Gly Lys Ile His Asn Asp Val Asn Ser Phe Thr
            20                  25                  30

Ile Asn Leu Gly Gln Gly Lys Glu Thr Leu Asn Leu His Phe Asn Pro
        35                  40                  45

Arg Phe Asn Glu Ser Thr Ile Val Cys Asn Thr Leu Asp Gly Ser Ser
    50                  55                  60

Trp Gly Gln Glu Gln Arg Glu Asn His Ser Cys Phe Ser Pro Gly Ser
65                  70                  75                  80

Glu Val Lys Leu Thr Leu Thr Phe Gln Asp Lys Asp Phe Lys Val Thr
                85                  90                  95

Leu Pro Asp Gly His Ser Leu Thr Phe Pro Asn Arg Leu Gly His Asn
            100                 105                 110

His Leu Arg Tyr Leu Ser Met Asp Gly Leu Gln Ile Ser Ser Phe Lys
        115                 120                 125

Leu Glu
    130

## Description

The present invention relates to novel uses of galectin-2.

Skin diseases affect approximately at least 20% of the world wide population. The human skin in an adult has an area of about 1.5 - 2 m² and represents the outside barrier between the body and the outside environment. It is subject to many influences by the exposure to a wide variety of media and agents. Accordingly, potential problems and disorders are manifold. In many skin disorders, an inflammation can be observed. Skin diseases with an inflammatory component are for example psoriasis, neurodermitis, acne, contact allergy, irritant dermatitis, autoimmune dermatitis, and lichen planus.

Inflammatory diseases of the skin are nowadays usually treated in a number of ways. For example, for the treatment of psoriasis, exposure to sun (UV-radiation), tar, sea water, and/or urea have proven useful in some cases. Moreover, drugs like corticosteroids or calcineurin inhibitors, such as tacrolimus, have shown some effect. However, in many instances, such anti-inflammatory drugs are associated with severe long-term side effects. For example corticosteroids may result in skin atrophy. Calcineurin inhibitors may be nephrotoxic and/or cause pruritus and erythema. In February 2005, the FDA has issued a warning for topically applied tacrolimus and stated that tacrolimus should only be used as a substitute drug if other drugs have failed.

Accordingly, it was an object of the present invention to provide for alternative ways of treating skin diseases with an inflammatory component.

The objects of the present invention are solved by the use of galectin-2 or of a nucleic acid coding for galectin-2 or of its complementary strand, or of a nucleic acid hybridizing to such coding nucleic acid or to its complementary strand, for the manufacture of a medicament for the treatment or prevention of the patient having an inflammatory disease of the skin.

In one embodiment said inflammatory disease of the skin is characterized by an abnormally increased level of activated effector-T-cells and/or an abnormally increased level of activated T-cells, said abnormally increased level being measured by flow cytometry.

In one embodiment said inflammatory disease of the skin is characterized by an abnormally increased level of activated CD8⁺ T-cells, as measured by flow cytometry.

Preferably, said inflammatory disease of the skin is selected from the group comprising psoriasis, eczema, atopic dermatitis, contact allergy, lichen planus and acne, wherein, more pref erably said inflammatory disease of the skin is selected from the group comprising atopic dermatitis and contact allergy.

The terms "atopic dermatitis", as used herein, refers to the same disease that is also sometimes referred to as "neurodermitis".

In one embodiment said galectin-2 is human or rat-galectin-2.

Preferably, said galectin-2 has an amino-acid sequence selected from the group comprising SEQ ID NO: 1 and SEQ ID NO: 2.

In one embodiment said galectin is administered in combination with an agent suppressing T-cell proliferation and/or an agent induced in T-cell apoptosis.

Preferably, said agent suppressing T-cell-proliferation is selected from the group comprising steroids, macrolides, such as cyclosporine and rapamaycin, tacrolimus, azathioprine, 6-mercaptopurine, methrotrexate and cyclophosphamide.

Preferably, said T-cell apoptosis inducing agent is selected from the group comprising anti-TNFα-antibody (infliximab, adalimumab and CDP 870), etanercept, leflunamide, natalizumab (anti-Integrin α4β7 mAb), visilizumab (anti-CD3 mAb).

In one embodiment said galectin-2 is administered in combination with anti-inflammatory drugs such as 5-aminosalicylate (5-ASA), corticosteroids, mesalazine, olsalazine, balsalazine, sulfapyridine and non-steroidal anti-inflammatory agents and/or antirheumatic agents, wherein, more preferably, said antirheumatic agent is a disease modifying anti rheumatic drug (DMARD), and wherein, more preferably, said disease modifying anti-rheumatic drug is selected from the group comprising diclofenac, ibuprofen, naprosyn, indomethacin, piroxican and biological drugs such as anakinra.

In one embodiment said galectin-2 is administered by systemical administration and/ or topical administration.

In one embodiment said galectin-2 is administered once to five times daily in an amount 0.25 mg to 2 mg/kg body weight per dose, preferably 0.75 mg to 1.5 mg/kg body weight per dose, most preferably about 1 mg/kg body weight per dose.

Preferably, said administration occurs by ingestion, preferably orally and/or by injection, preferably by intravenous, intramuscular, intraperitoneal or subcutaneous injection, and/or by nasal application.

Preferably, said galectin-2 is administered in a pegylated or non-pegylated form or as a mixture of the two forms.

The inventors have surprisingly found that galectin-2 may be used to treat inflammatory skin diseases. They could show that in inflammatory skin diseases where there is an increased level of activated T-cells such as contact allergy or psoriasis, galectin-2 could be successfully used to lower the level of activated T-cells and/or to prevent an increase in the level of activated T-cells. It is therefore to be expected that galectin-2 is useful in particular for atopic dermatitis, contact allergy, psoriasis, lichen planus and acne

Galectins are a family of related animal lectines having highly conserved recognition sequences for beta-galactosidase. Galectins are expressed in different cells of the immune system. In the human and the murine system, 15 galectins have been described with manifold functions. It could for example be shown that galectins induce cell activation and apoptosis. Moreover, they have been shown to modulate cell adhesion and cell migration and to influence the cell growth by regulating the cell cycle. Some members of the galectin-family have anti-inflammatory characteristics, whereas others are known to be pro-inflammatory and play a significant role in acute or chronic inflammations. Hence, what applies to one member of the galectin-family does not necessarily apply to other members as well.

Galectin-2 (Gal-2) was discovered during the cloning of galectin-1 and has 43% sequence identity to human galectin-1, revealing the greatest homology with galectin-1 among all others galectins examined. Galectin-2 is a non-covalent dimer with subunits of about 14 kDa. Galectin-2 is also known as beta galactoside binding lectin, Lectin I 14, LGALS2, Gal-2 or GAL-2. Expression of galectin-2 seems to be restricted to the gastrointestinal (GI) tract. It is also known that intestinal epithelial cells that express galectin-2 do not normally express galectin-1 and that galectin-2 is not expressed at elevated levels in galectin-1 null mutant mice. Whereas the properties of galectin-1 have been studied more extensively, the function of galectin-2 has not been studied so far and thus remains unclear until now.

As used herein, the term "galectin-2" is meant to designate a protein as encoded by SEQ ID NO: 1 or SEQ ID NO: 2 and proteins having more than 45%, preferably more than 60%, preferably more than 80%, more preferably more than 90% sequence identity thereto (i. e. over 45, 60, 80 or 90%, respectively, of the entire length of SEQ ID NO: 1 or SEQ ID NO: 2). It also is meant to signify any functional variant thereof which shows a selective induction of T-cell apoptosis in activated T-cells.

The term "galectin-2 is administered ...", as used herein, is meant to signify the it may be administered as a protein or as the nucleic acid encoding galectin-2-protein and/or as the nucleic acid strand complementary to the coding nucleic acid. In a preferred embodiment, it is meant to signify that galectin-2 be administered as a protein.

The term "... is administered in combination with ...", as used herein is meant to signify that galectin-2 may be administered in conjunction with other agents. The administration may be simultaneous or one after the other, it may be via the same route or via different routes, it may be in the same dosage form or via separate dosage forms.

The term "an abnormally increased level of activated effector-T-cells" and the term "an abnormally increased level of activated T-cells", as used herein, is meant to refer to any state wherein the level of the respective cells within the organism, such as a human being, is significantly increased in comparison to a healthy state of the organism. Levels of cells can be conveniently measured by for example flow cytometrical methods which are well known to someone skilled in the art. These flow cytometrial methods can be performed in conjunction with proliferation assays, wherein specific parameters are varied, such as cytokine/mediator production, expression or suppression of transcription factors or surface marker expression that are known to be associated with function.

Administration of galectin-2 may be in any form suitable for the recipient, provided that an uptake of galectin-2 into the recipient's organism is ensured. Administration may additionally be in combination with any pharmaceutically acceptable carrier. It may be systemic and/or topical, it may be injected, preferably intravenously, intramuscularly, intraperitoneally and/or subcutaneously, or it may be applied via other routes, e. g. as enema or suppository or via nasal application.

Galectin-2, in particular human galectin-2, is not immunogenic and therefore is particularly well suited for application as a medicament in human patients.

More specifically, the inventors could show that the administration of galectin-2 in a murine model of an inflammatory disease significantly inhibited the inflammatory disease and reduced an inflammatory allergic reaction in this model. This suppressive effect could also be detected when the inflammatory disease was triggered a second time in sensitized animals. This shows that using galectin-2 enables to treat skin diseases with an inflammatory component.

Moreover, reference is made to the figures, wherein
figure 1 shows a reduced contact allergy reaction in mice treated with galectin-2,
figure 2 shows the reduced numbers of CD8⁺ T-cells in cervical lymph nodes of mice treated with galectin-2,
figure 3 shows the induction of apoptosis in CD8⁺ T-cells by means of a system treatment using galectin-2.

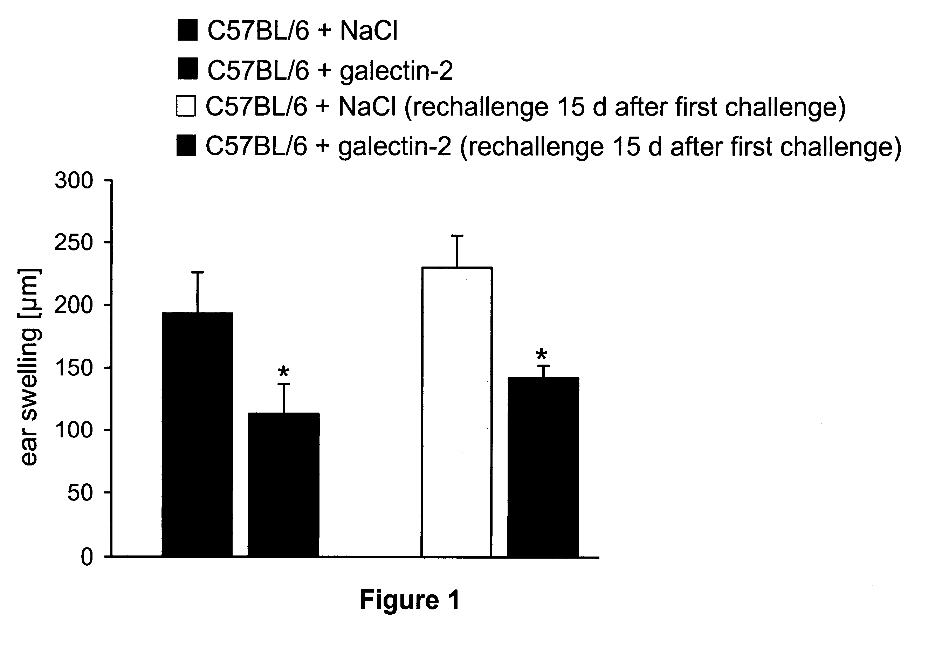

More specifically, figure 1 shows reduced contact hypersensitivity (CHS) responses in mice treated with galectin-2. Animals were sensitized with DNFB and DNFB ear challenged.

Groups of mice were injected intraperitoneally with 20 µg galectin-2 on 6 consecutive days prior to first ear challenge. Ear swelling as a measure of contact hypersensitivity responses was assessed 48 h after challenge. Data are shown as mean ear swelling ± SD and are representative of 10 mice. The * indicates statistical significance (Student's t-test; *p* < 0.05) between CHS responses in NaCl and galectin-2 treated mice.

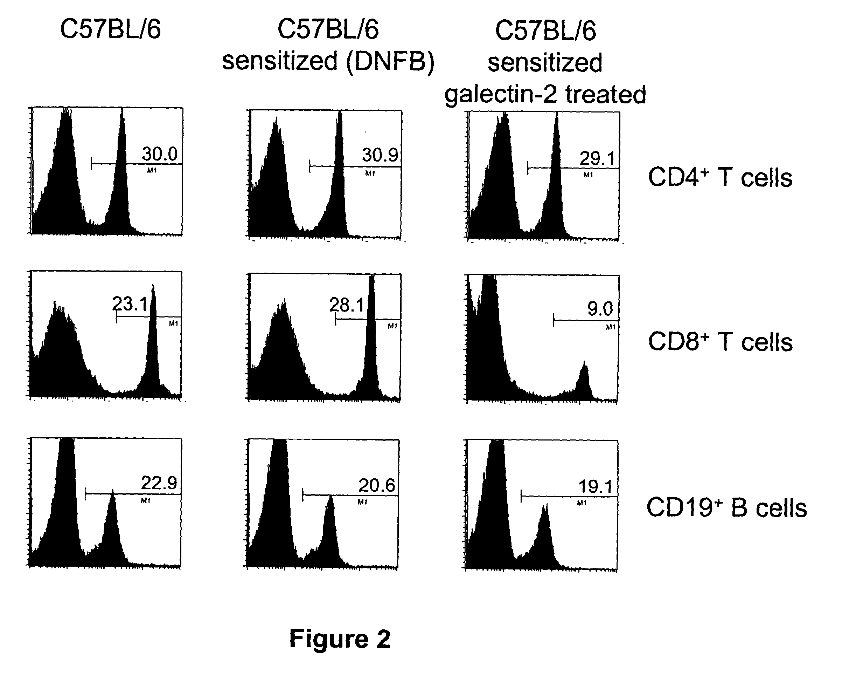

More specifically, figure 2 shows reduced numbers of CD8+ T cells in cervical lymph nodes of galectin-2 treated mice. C57BL/6 mice were injected intraperitoneally with 20 µg galectin-2 on 6 consecutive days. At day 7 mice were epicutaneously sensitized with DNFB on the shaved back and at day 12 cell populations in cervical lymph nodes were analyzed by multicolor flow cytometry.

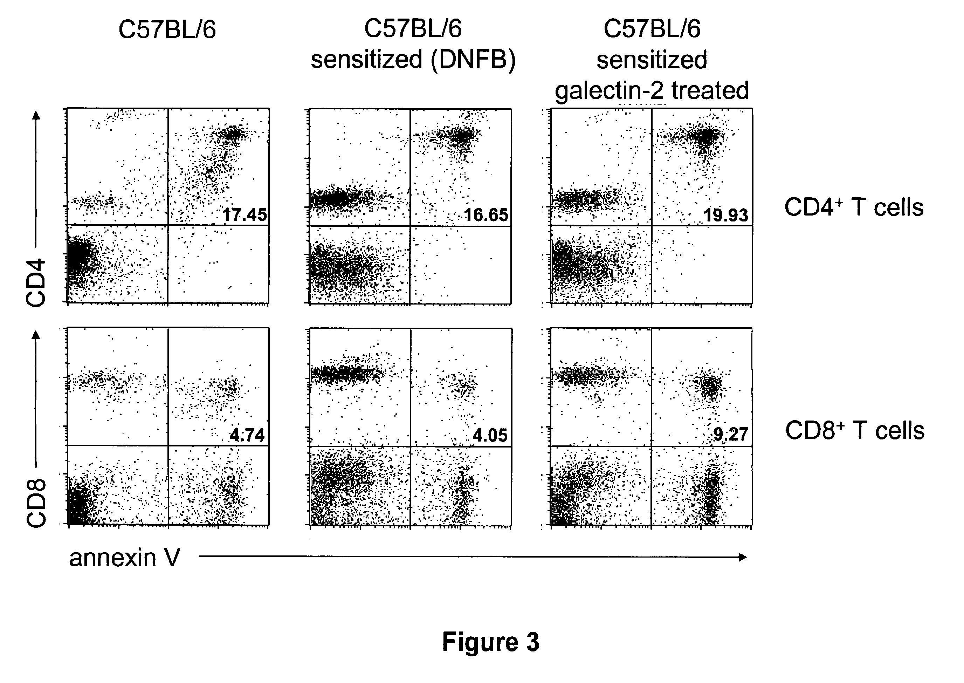

More specifically, figure 3 shows systemic galectin-2 treatement induces apoptosis in CD8+ T cells. Cervical lymph nodes of naive C57BL/6 mice, sensitized mice as well as galectin-2 treated and sensitized mice were analyzed by flow cytometry showing increased numbers of annexin V expressing CD8+ T cells in galectin-2 treated mice.

Moreover, reference is made to the sequence listing to this application, wherein
SEQ ID NO: 1 is the amino acid sequence of human galectin-2, and
SEQ ID NO: 2 is the sequence of galectin-2 from rat.

Furthermore, reference is made to the following example which is given to illustrate, not to limit the present invention.

### Example

Galectin-2 from human and rat was prepared as described in WO 2005/092368 which is incorporated herein by reference in its entirety.

In a murine model of contact allergy the inventors examined whether the systemic administration of galectin-2 led to a suppression of the contact allergic reaction. Wild type mice were treated systemically with 20 µg galectin-2 each on 6 consecutive days and were subsequently sensitized using the hapten dinitrofluorobenzene (DNFB). Thereafter, the contact allergy reaction was triggered by applying DNFB at the ear. The sensitization occurred on the shaved backs of the animal. The systemical treatment using galectin-2 was by intraperitoneal injection of galectin-2. As can be seen from figure 1, the animals treated with galectin-2 had a significantly reduced contact allergic reaction in comparison with the control mice which only treated with saline vehicle (NaCl). Figure 1 shows the swelling of the ear lobe in response to the triggering of the contact allergy. More specifically, mice were sensitized by painting 100 µl of 0.5 % DNFB, in acetone/olive oil (4/1) on the shaved back. For elicitation of CHS responses, 12 µl of 0.3 % DNFB were painted on both sides of the left ear on day five. CHS was determined by the degree of ear swelling of the hapten-exposed left ear compared to the ear thickness of the not-challenged right ear and measured with a micrometer (Mitutoyo, Tokyo, Japan) at indicated time points after challenge. Mice that were ear challenged without prior sensitization served as negative controls.

The result obtained means that the systemic administration of galectin-2 leads to an inhibition of the inflammatory allergic reaction. More interestingly, this suppressive effect could also be detected when the contact allergy reaction was triggered a second time in the sensitized animals, 15 days after the end of galectin-treatment. *p < 0,05 vs. NaCl-treatment. (see figure 1)

The inventors furthermore examined the cervical lymph nodes of mice that have been sensitized with DNFB and treated with galectin-2 or NaCl. In a flow cytometric analysis the number of CD8⁺ T-cells in the lymph nodes of galectin-2 treated mice were significantly reduced in comparison to the control animals treated with NaCl only, whereas the number of CD4+ T-cells as well as the other leukocyte subpopulations, such as B-cells remained unchanged, as can be seen in figure 2. For the experiment of figure 2, naive C57BL/6 mice were intraperitoneally injected with 20 µg galectin-2 on 6 consecutive days. On day 7, the animals were sensitized with DNFB, and on day 12, the cervical lymph nodes were isolated and flow-cytometrically analyzed.

In the increased CD8⁺ T-cell population, an increased annexine-V-expression could be detected which suggests that the systemic treatment using galectin-2 specifically induces the apoptosis of activated CD8⁺ T-cells. This can be seen in figure 3 showing flow cytometric analysis of cervical lymph nodes from sensitized mice that were treated with NaCl or galectin-2. An increased expression of annexin-V in CD8⁺ T-cells can be observed in galectin-2 treated animals. The x-axis shows increasing levels of annexin-V⁺ cells.

Hence, without wishing to be bound, the present inventors believe that the use of galectin-2 leads to an apoptosis of activated pro-inflammatory CD8⁺ T-cells in the inflammation areas, whereas the remaining leukocyte sub-populations remained unaffected. The systemical or topical application thus leads to a reduction of activity and proliferation of CD8⁺ T-cells which are significantly involved in the triggering of an inflammatory reaction of the skin, such as is encountered in contact allergic reactions or atopic dermatitis. Therefore, the use of galectin-2 is a very effective alternative for a therapy of acute and chronic inflammatory skin diseases, whilst having little or no side effects.
The features of the present invention disclosed in the specification, the claims and/or in the accompanying drawings, may, both separately, and in any combination thereof, be material for realizing the invention in various forms thereof.

## Claims

1. Use of galectin-2 or of a nucleic acid coding for galectin-2 or of its complementary strand, or of a nucleic acid hybridizing to such coding nucleic acid or to its complementary strand, for the manufacture of a medicament for the treatment or prevention of the patient having an inflammatory disease of the skin.

2. Use according to claim 1, wherein said inflammatory disease of the skin is **characterized by** an abnormally increased level of activated effector-T-cells and/or an abnormally increased level of activated T-cells, said abnormally increased level being measured by flow cytometry.

3. Use according to any of claims 1 - 2, wherein said inflammatory disease of the skin is **characterized by** an abnormally increased level of activated CD8⁺ T-cells, as measured by flow cytometry.

4. Use according to any of the foregoing claims, wherein said inflammatory disease of the skin is selected from the group comprising psoriasis, eczema, atopic dermatitis, contact allergy, lichen planus and acne.

5. Use according to claim 4, wherein said inflammatory disease of the skin is selected from the group comprising atopic dermatitis and contact allergy.

6. Use according to any of the foregoing claims, wherein said galectin-2 is human or rat-galectin-2.

7. Use according to any of the foregoing claims, wherein said galectin-2 has an amino-acid sequence selected from the group comprising SEQ ID NO: 1 and SEQ ID NO: 2.

8. Use according to any of the foregoing claims, wherein said galectin is administered in combination with an agent suppressing T-cell proliferation and/or an agent induced in T-cell apoptosis.

9. Use according to claim 8, wherein said agent suppressing T-cell-proliferation is selected from the group comprising steroids, macrolides, such as cyclosporine and rapamaycin, tacrolimus, azathioprine, 6-mercaptopurine, methrotrexate and cyclophosphamide.

10. Use according to claim 8, wherein said T-cell apoptosis inducing agent is selected from the group comprising anti-TNFα-antibody (infliximab, adalimumab and CDP 870), etanercept, leflunamide, natalizumab (anti-Integrin *α*4*β*7 mAb), visilizumab (anti-CD3 mAb).

11. Use according to any of the foregoing claims, wherein said galectin-2 is administered in combination with anti-inflammatory drugs such as 5-aminosalicylate (5-ASA), corticosteroids, mesalazine, olsalazine, balsalazine, sulfapyridine and non-steroidal anti-inflammatory agents and/or antirheumatic agents.

12. Use according to claim 11, wherein said antirheumatic agent is a disease modifying anti rheumatic drug (DMARD).

13. Use according to claim 12, wherein said disease modifying anti-rheumatic drug is selected from the group comprising diclofenac, ibuprofen, naprosyn, indomethacin, piroxican and biological drugs such as anakinra.

14. Use according to any of the foregoing claims, wherein said galectin-2 is administered by systemical administration and/ or topical administration.

15. Use according to any of the foregoing claims, wherein said galectin-2 is administered once to five times daily in an amount of 0.25 mg to 2 mg/kg bodyweight per dose, preferably 0.75 mg to 1.5 mg/kg body weight per dose, most preferably about 1 mg/kg bodyweight per dose.

16. Use according to any of claims 14 - 15, wherein said administration occurs by ingestion, preferably orally and/or by injection, preferably by intravenous, intramuscular, intraperitoneal or subcutaneous injection, and/or by nasal application.

17. Use according to any of claims 14 - 16, wherein said galectin-2 is administered in a pegylated or non-pegylated form or as a mixture of the two forms.
